# EUROPÄISCHE PATENTSCHRIFT

(11) **EP 2 483 387 B1**
(45) Veröffentlichungstag und Bekanntmachung des Hinweises auf die Patenterteilung: **16.05.2018**
(21) Anmeldenummer: 10762905.7
(22) Anmeldetag: 29.09.2010
(51) Int. Cl.: C12M 1/34

(54) **VERFAHREN UND ANORDNUNG ZUR BESTIMMUNG VON ZELL-VITALITÄTEN**
METHOD AND ASSEMBLY FOR DETERMINING CELL VITALITIES
PROCÉDÉ ET ENSEMBLE DE DÉTERMINATION DE LA VITALITÉ CELLULAIRE

(30) Priorität: 30.09.2009 DE 102009043537
(43) Veröffentlichungstag der Anmeldung: 08.08.2012
(73) Patentinhaber: Siemens Aktiengesellschaft, 80333 München (DE)
(72) Erfinder: GUMBRECHT, Walter, 91074 Herzogenaurach (DE); KLÖPZIG, Markus, 91320 Ebermannstadt (DE); SCHMIDT, Harald, 91058 Erlangen (DE); PAULICKA, Peter, 91341 Röttenbach (DE); STANZEL, Manfred, 92334 Berching (DE)
(86) Internationale Anmeldenummer: PCT/EP2010/064483
(87) Internationale Veröffentlichungsnummer: WO 2011/039271

(56) Entgegenhaltungen:
- WO-A2-03/012447
- US-A1- 2002 166 764
- US-A1- 2004 018 611
- VARSHNEY M ET AL: "Interdigitated array microelectrodes based impedance biosensors for detection of bacterial cells", BIOSENSORS AND BIOELECTRONICS, Bd. 24, Nr. 10, 15. Juni 2009 (2009-06-15) , Seiten 2951-2960, XP026119026, ISSN: 0956-5663, DOI: DOI:10.1016/J.BIOS.2008.10.001
- VARSHNEY M ET AL: "Interdigitated array microelectrode based impedance biosensor coupled with magnetic nanoparticle-antibody conjugates for detection of Escherichia coli O157:H7 in food samples", BIOSENSORS AND BIOELECTRONICS, Bd. 22, Nr. 11, 30. März 2007 (2007-03-30) , Seiten 2408-2414, XP022006434, ISSN: 0956-5663, DOI: DOI:10.1016/J.BIOS.2006.08.030
- VARSHNEY M ET AL: "A label-free, microfluidics and interdigitated array microelectrode-based impedance biosensor in combination with nanoparticles immunoseparation for detection of Escherichia coli O157:H7 in food samples", SENSORS AND ACTUATORS B, Bd. 128, Nr. 1, 7. November 2007 (2007-11-07), Seiten 99-107, XP022335649, ISSN: 0925-4005, DOI: DOI:10.1016/J.SNB.2007.03.045
- YANG L ET AL: "Detection of viable Salmonella using microelectrode-based capacitance measurement coupled with immunomagnetic separation", JOURNAL OF MICROBIOLOGICAL METHODS, Bd. 64, Nr. 1, 1. Januar 2006 (2006-01-01) , Seiten 9-16, XP025072993, ISSN: 0167-7012, DOI: DOI:10.1016/J.MIMET.2005.04.022 [gefunden am 2006-01-01]
- GEHRING A G ET AL: "Enzyme-linked immunomagnetic electrochemical detection of Salmonella typhimurium", JOURNAL OF IMMUNOLOGICAL METHODS, Bd. 195, Nr. 1, 9. September 1996 (1996-09-09), Seiten 15-25, XP004021249, ISSN: 0022-1759, DOI: DOI:10.1016/0022-1759(96)00076-2

## Beschreibung

Die vorliegende Erfindung bezieht sich auf ein Verfahren und eine Anordnung zur Bestimmung von Zell-Vitalitäten. Das Verfahren umfasst die Schritte, Binden von lebenden Zellen an magnetische Partikel, Aufbringen auf ein Sensor-Array, gleichmäßige Verteilung über dem Sensor-Array, magnetische Fixierung der magnetischen Partikel mit den gebundenen Zellen über dem Sensor-Array, und Aufbringen von Substanzen zur Aufrechterhaltung und/oder Verbesserung der Zell-Vitalität auf das Sensor-Array, und/oder Aufbringen von Substanzen zur Verschlechterung der Zell-Vitalität auf das Sensor-Array, wobei das Sensor-Array mit einer geschlossenen Schicht von magnetischen Partikeln beschichtet wird, bevor eine Schicht mit lebenden Zellen auf dem Sensor-Array gebildet wird, wobei Hohlräume zwischen den magnetischen Partikeln der geschlossenen Schicht mit Flüssigkeit befüllbar sind. Die Anordnung umfasst ein Sensor-Array aufgebaut aus Sensoren, welche ausgebildet sind mit einem Fluid in direktem fluidischen Kontakt zu stehen, und eine Einrichtung zum Erzeugen eines Magnetfeldes über dem Sensor-Array, dadurch gekennzeichnet, dass auf dem Sensor-Array eine Schicht ausgebildet ist, welche magnetische Partikel und lebende Zellen umfasst, und dass zwischen der Schicht mit den lebenden Zellen und den Sensoren des Sensor-Arrays wenigstens eine geschlossene Schicht von magnetischen Partikeln angeordnet ist, wobei Hohlräume zwischen den magnetischen Partikeln der geschlossenen Schicht mit Flüssigkeit befüllbar sind.

In der Mikrobiologie sind eine Vielzahl von Methoden zur Untersuchung von pathogenen Keimen auf der Basis von Zellkultur- und Antibiotika-Resistenztests bekannt. Von Vorteil ist der "phänotypische" Ansatz, bei welchem die Wirkung, wie z.B. das Wachstum bzw. die Hemmung des Zellwachstums, untersucht wird. Über die Wirkung auf Zellkulturen kann ein direkter Bezug zur Wirkung auf Menschen oder Tieren gewonnen werden.

Zellkulturen werden dabei in einer Nährlösung über Tage hinweg z.B. in Petrischalen eingelegt und beobachtet. Das Wachstum bzw. die Schädigung der Zellkulturen wird über lange Zeiträume hinweg gemessen und ausgewertet. Die langen Zeiträume, welche für die Beobachtung notwendig sind, machen das Verfahren sehr aufwändig und langwierig.

Zur Messung des Wachstums bzw. der Schädigung der Zellkulturen können Sensorsysteme verwendet werden. Lebende Zellen werden z.B. auf Sensoren aufgewachsen, um danach die Vitalität der Zellen z.B. durch Messung von Impedanz, Sauerstoff oder pH-Wert zu überwachen. Als Sensoren können Interdigitalelektroden-Arrays, Sauerstoff-Sensoren oder pH-Wert-Sensoren verwendet werden. Maße für die Vitalität der Zellen sind unter anderem deren Adhäsion auf Oberflächen, deren Atmung oder deren Stoffwechsel. Das Aufwachsen der Zellen auf den Sensoren ist jedoch zeitaufwändig und führt zu einer eingeschränkten Lagerfähigkeit der Sensorsysteme. Aufgewachsene Zellen auf den Sensoren können auf der Oberfläche wandern und/oder absterben.

Für eine Messung der Vitalität von Zellen über einen Sauerstoff-Wert oder pH-Wert ist ein definierter Flüssigkeitsfilm zwischen einer Zellwand und einer Sensor-Oberfläche notwendig. Bei einem unmittelbaren Aufwachsen der Zellwand auf der Sensor-Oberfläche kann der definierte Flüssigkeitsfilm verloren gehen. Dies kann zu einer Beeinträchtigung der Messung führen bis hin zu dem Fall, bei welchem eine Messung unmöglich wird.

Für eine zuverlässige Messung ist es weiterhin notwendig, dass die Sensor-Oberfläche frei von abgestorbenen Zellen ist. Aus diesem Grund müssen vor jedem Behandlungs- bzw. Messintervall abgestorbene Zellen von der Sensor-Oberfläche entfernt werden. Dies erfolgt in der Regel mit Hilfe von Reagenzien, was mit Aufwand verbunden ist und zu einer Schädigung der Sensor-Oberflächen führen kann. Vergleichbare und reproduzierbare Messungen werden dadurch verhindert.

Die WO 03/012447 A2 beschreibt ein Verfahren für den Nachweis lebender Zellen mittels magnetisierbarer Partikel, an die die Zellen gebunden werden, und die dann mit einem Magneten lokal konzentriert werden. Dabei misst ein Sensor eine sich stoffwechselabhängig verändernde Stoffkonzentration, wie zum Beispiel eine Sauerstoffkonzentration.

Aufgabe der vorliegenden Erfindung ist es, ein Verfahren und eine Anordnung zur Bestimmung von Zell-Vitalitäten anzugeben, welche eine schnelle und einfache sowie zuverlässige Messung von Parametern erlauben, die typisch für die Zell-Vitalität sind. Dabei sollen Fehlerfaktoren, wie z.B. das Wandern von Zellen auf der Oberfläche oder Messfehler durch ein direktes Aufwachsen ohne Flüssigkeitsfilm zwischen Zelle und Sensor-Oberfläche ausgeschlossen sein.

Die angegebene Aufgabe wird bezüglich des Verfahrens zur Bestimmung von Zell-Vitalitäten durch die Merkmale des Anspruchs 1 und bezüglich der Anordnung zur Bestimmung von Zell-Vitalitäten durch die Merkmale des Anspruchs 9 gelöst.

Vorteilhafte Ausgestaltungen des erfindungsgemäßen Verfahrens und der Anordnung zur Bestimmung von Zell-Vitalitäten gehen aus den jeweils zugeordneten abhängigen Unteransprüchen hervor. Dabei können die Merkmale des Hauptanspruchs mit Merkmalen der Unteransprüche und Merkmale der Unteransprüche untereinander kombiniert werden.

Das erfindungsgemäße Verfahren zur Bestimmung von Zell-Vitalitäten umfasst die Schritte, Binden von lebenden Zellen an magnetische Partikel, und Aufbringen der magnetischen Partikel mit gebundenen Zellen auf einem Sensor-Array, und gleichmäßige Verteilung der magnetischen Partikel mit den gebundenen Zellen über dem Sensor-Array, und magnetische Fixierung der magnetischen Partikel mit den gebundenen Zellen über dem Sensor-Array, und Aufbringen von Substanzen zur Aufrechterhaltung und/oder Verbesserung der Zell-Vitalität auf das Sensor-Array, wobei das Sensor-Array mit einer geschlossenen Schicht von magnetischen Partikeln beschichtet wird, bevor eine Schicht mit lebenden Zellen auf dem Sensor-Array gebildet wird, wobei Hohlräume zwischen den magnetischen Partikeln der geschlossenen Schicht mit Flüssigkeit befüllbar sind. Weiterhin kann das Aufbringen von Substanzen zur Verschlechterung der Zell-Vitalität auf das Sensor-Array umfasst sein.

Durch das Binden der lebenden Zellen an magnetische Partikel werden die Zellen durch ein äußeres Magnetfeld in ihrer Bewegung steuerbar. Sie können z.B. durch Antikörper an die magnetischen Partikel gebunden werden, insbesondere wenn die Partikel einen Durchmesser im Bereich von Nano- bis Mikrometern aufweisen. Bei größeren Partikeln im Bereich von einigen hundert Mikrometern Durchmesser können die Zellen auch auf der Oberfläche der Partikel aufwachsen. Nach dem Binden können die Zellen in einem Vorratsbehälter zwischengespeichert werden. Zur Vermessung der Zell-Vitalität der lebenden Zellen werden die Zellen dann über zu einem Sensor-Array bewegt und dort magnetisch fixiert. Die Bewegung kann z.B. durch eine strömende Flüssigkeit oder durch magnetische Wechselwirkung erfolgen. Magnetisch über dem Sensor-Array fixierte Zellen können dann vermessen werden, wozu Substanzen zur Aufrechterhaltung und/oder Verbesserung und/oder Verschlechterung der Zell-Vitalität dienen. Die chemischen Produkte, welche beim Stoffwechsel der Zellen entstehen oder der Verbrauch von chemischen Substanzen beim Stoffwechsel der Zellen werden mit Hilfe der Sensoren des Sensor-Arrays gemessen, z.B. qualitativ und/oder quantitativ. Aufgrund der Anordnung der Sensoren in Form eines Arrays können diese Messungen räumlich aufgelöst erfolgen.

Die Verwendung von magnetischen Partikeln zum Handeln der Zellen erlaubt einen bedarfsgerechten Einsatz von Zellen und eine schnelle, zuverlässige Zuführung lebender Zellen zum Sensor-Array. So können z.B. zuvor präparierte, in dem Vorratsbehälter gespeicherte lebende Zellen bei zu erfolgender Messung z.B. von Umweltgiften dem Sensor-Array zeitnah zugeführt werden. Alternativ können z.B. aus Blut bestimmte Zellen durch Binden an die magnetischen Partikel "herausgefiltert" werden und mit Hilfe der magnetischen Partikel gezielt dem Sensor-Array zugeführt werden. Dies kann einfacher und kostengünstiger geschehen als z.B. durch Handling mit Hilfe von Pipetten oder durch Vermehren von Zellkulturen über Tage hinweg auf bestimmten Nährlösungen.

Die Substanzen zur Aufrechterhaltung und/oder Verbesserung der Zell-Vitalität können Sauerstoff und/oder Nährlösung umfassen. Die Substanzen zur Verschlechterung der Zell-Vitalität können Antibiotika umfassen. Die Substanzen können bei einer Messung gezielt einmalig oder abwechselnd in Intervallen auf das Sensor-Array gegeben werden, und Änderungen in Stoffwechselprodukten der Zellen können dabei gemessen werden. Dies erlaubt zuverlässige und schnelle Aussagen über die Zell-Vitalität und ist kostengünstiger und zeitsparender als die Beobachtung von Zellwachstum einzelner Zellkulturen in Nährlösungen in Petrischalen z.B. optisch.

In einem Schritt des Verfahrens kann eine optimale Temperatur für Zell-Vitalität eingestellt werden, insbesondere 37°C. Bei dieser Temperatur ist das Messsignal von umgesetzten Stoffwechselprodukten oder die Abnahme von Ausgangssubstanzen der Stoffwechselreaktionen der Zellen besonders groß und somit einfach zu messen.

Die Sensoren des Sensor-Arrays können elektrochemische und/oder chemische Sensoren umfassen. Diese können insbesondere ausgelegt sein Messwerte zu messen, welche als Maß für die Zell-Vitalität dienen. Bei elektrochemischen Messungen im Gegensatz zu optischen Messungen stören die nicht transparenten magnetischen Partikel die Messung nicht. Elektrochemische Sensoren können sehr klein und kostengünstig in Array-Form hergestellt werden und liefern zuverlässige Messergebnisse. Die rein elektrische Auswertung von Strom-Spannungs-Signalen von elektrochemischen Sensoren ist einfacher und kostengünstiger zu realisieren, als z.B. bei optischen Messungen.

Als Messgrößen der Sensoren können von Zellen konsumierte Stoffe und/oder Stoffwechselprodukte von Zellen gemessen werden, insbesondere Säuren als pH-Wert und/oder Sauerstoff als pO₂-Wert und/oder Proteine. Diese Größen sind eindeutige Messgrößen für die Vitalität einer Zelle. So wird z.B. durch den Stoffwechsel einer Zelle Sauerstoff in ihrer Umgebung umgesetzt. Die Abnahme des Sauerstoffs in ihrer direkten Umgebung ist somit ein eindeutiges Maß für die Vitalität der Zelle.

Die an die magnetischen Partikel gebundenen Zellen über dem Sensor-Array können bei Bedarf entfernt werden, insbesondere über eine Manipulation des Magnetfeldes über dem Array. Dadurch kann ein Sensor wieder regeneriert und für die nächste Messung vorbereitet werden. Ein über längere Zeiträume hinweg intervallartiges Messen wird so ermöglicht. Tote Zellen würden ohne sie zu entfernen, die Sensoren blockieren und Messergebnisse verfälschen oder eine Messung ganz unmöglich machen. Durch das Messen mit Hilfe des Sensor-Arrays können tote Zellen identifiziert werden und über das Magnetfeld gezielt abtransportiert werden. Gerade in Hinblick auf das Messen von Umweltgiften und die Funktion eines Sensors über längere Zeit hinweg, ist ein Abtransport von toten Zellen und eine dadurch erfolgende Möglichkeit der Erneuerung des Sensors sinnvoll.

Dabei können dem Schritt des Entfernens von toten Zellen die Schritte Binden von lebenden Zellen an magnetische Partikel, Aufbringen der magnetischen Partikel mit gebundenen Zellen auf dem Sensor-Array, und gleichmäßige Verteilung der magnetischen Partikel mit den gebundenen Zellen über dem Sensor-Array für eine zuverlässige, gezielte Messung einzelner Zellen, und magnetische Fixierung der magnetischen Partikel mit den gebundenen Zellen über dem Sensor-Array, und Aufbringen von Substanzen zur Aufrechterhaltung und/oder Verbesserung der Zell-Vitalität auf das Sensor-Array, und/oder Aufbringen von Substanzen zur Verschlechterung der Zell-Vitalität auf das Sensor-Array, wiederholt folgen, wobei das Sensor-Array mit einer geschlossenen Schicht von magnetischen Partikeln beschichtet wird, bevor eine Schicht mit lebenden Zellen auf dem Sensor-Array gebildet wird. Dadurch wird eine Messung über lange Zeiträume oder eine wiederholte Verwendung eines Sensor-Arrays für verschiedene Messungen möglich.

Die erfindungsgemäße Anordnung zur Bestimmung von Zell-Vitalitäten umfasst ein Sensor-Array, aufgebaut aus Sensoren, welche ausgebildet sind mit einem Fluid in direktem fluidischen Kontakt zu stehen, und eine Einrichtung zum Erzeugen eines Magnetfeldes über dem Sensor-Array. Auf dem Sensor-Array ist eine Schicht ausgebildet, welche magnetische Partikel und lebende Zellen gleichzeitig umfasst. Zwischen der Schicht mit den lebenden Zellen und den Sensoren des Sensor-Arrays ist wenigstens eine geschlossene Schicht von magnetischen Partikeln angeordnet, wobei Hohlräume zwischen den magnetischen Partikeln der geschlossenen Schicht mit Flüssigkeit befüllbar sind. Die Anordnung kann für das zuvor beschriebene Verfahren verwendet werden.

Dabei können die lebenden Zellen in einer Matrix von magnetischen Partikeln in der Schicht auf dem Sensor-Array eingebettet sein. Dies stellt sicher, dass wenigstens einige oder alle Zellen nicht direkt auf den Sensoren aufwachsen und ein Flüssigkeitsfilm zwischen den Zellen und den Sensoren angeordnet ist. Dies macht eine zuverlässige Messung der Vitalität der Zellen erst möglich. Ohne einen Flüssigkeitsfilm zwischen den Zellen und der Sensor-Oberfläche ist eine zuverlässige Registrierung der Reaktionsprodukte oder Ausgangsstoffkonzentration bzw. deren Änderung durch die Sensoren nicht möglich. Die magnetischen Partikel dienen sozusagen als Abstandshalter für die Zellen, um ein direktes Aufwachsen aller Zellen auf den Sensor-Oberflächen zu verhindern.

Die Schicht auf dem Sensor-Array, umfassend magnetische Partikel und lebende Zellen, kann eine im Wesentlichen gleiche Dicke über dem Bereich des Sensor-Arrays aufweisen. Insbesondere kann die Dicke der Schicht im Bereich von Mikrometern liegen. Ganz besonders bevorzugt kann die Dicke der Schicht im Bereich von 10 bis 1000 Mikrometern liegen. Durch eine gleichmäßige Schichtdicke wird eine Häufung von Zellen über einzelnen Sensoren verhindert und bei einer Dicke im Bereich von Mikrometern befinden sich Zellen in der Partikel-Matrix nah genug an einem nächsten Sensor, bei entsprechend geringem Abstand der Sensoren voneinander, damit Produkte oder die Abnahme von Ausgangssubstanzen des Zell-Stoffwechsels von den Sensoren registriert werden können. Mit im Wesentlichen gleiche Dicke ist gemeint, dass gewisse Schwankungen aufgrund der Unebenheiten von runden Partikeln und Welligkeiten in der Schichtoberfläche durch geringe Schwankungen von Partikelzahlen an einer Stelle im Bereich von weniger als einer Zehnerpotenz möglich sind.

Zwischen den lebenden Zellen und den Sensoren des Sensor-Arrays ist wenigstens eine geschlossene Schicht von magnetischen Partikeln angeordnet, was sicher stellt, dass keine Zellen direkt auf einer Sensor-Oberfläche aufwachsen. Hohlräume zwischen den magnetischen Partikeln sind mit Flüssigkeit befüllbar, um elektrochemische Messungen zu ermöglichen. Das Sensor-Array wird mit einer geschlossenen Schicht von magnetischen Partikeln beschichtet, bevor die Schicht mit lebenden Zellen auf dem Sensor-Array gebildet wird. Eine Trennung der Gesamtschichtbildung in zwei Schritte erhöht die Zuverlässigkeit der Vitalitätsmessung der Zellen, da sicher gestellt ist, dass sich keine Zellen in die geschlossene Schicht einlagern und direkt auf einer Sensor-Oberfläche aufwachsen.

Die Anordnung kann eine Durchflusszelle mit einem Träger umfassen, wobei auf einer Oberfläche des Trägers in fluidischer Verbindung mit der Durchflusszelle das Sensor-Array angeordnet ist.

Die Sensoren des Sensor-Arrays können elektrochemische Sensoren sein, insbesondere Mikrosensoren mit einem Gesamtplatzverbrauch eines Sensors an der Oberfläche des Sensor-Arrays im Bereich von Mikrometern. Damit ist die Größenordnung eines Sensors im Bereich einer Zelle und eine Zuordnung des Messsignals eines Sensors zu einer Zelle wird möglich. Der Umsatz an Substanzen einer Zelle liegt in einem Bereich, welcher von einem Sensor mit einer Größe im Mikrometerbereich gemessen werden kann. Sensoren, welche viel größer sind, z.B. in der Größenordnung von Millimetern, können Konzentrationsänderungen in einem so geringem Maß, wie sie vom Zellstoffwechsel ausgelöst werden in der direkten Umgebung einer Zelle, nicht zuverlässig messen. Die Verwendung von elektrochemischen Sensoren macht eine Ausbildung der Sensoren im Bereich von Mikrometern erst möglich und liefert auch bei nicht transparenten Partikeln zuverlässige Messsignale.

Die Anordnung kann wenigstens eine Einrichtung zum Ändern des Magnetfeldes umfassen. Diese kann eine Spuleneinrichtung und/oder eine Einrichtung zum Bewegen von Permanentmagneten sein. Dadurch kann das Magnetfeld so ausgebildet werden, dass eine gleichmäßige Schicht an Magnetpartikeln über dem Sensor-Array ausgebildet wird. Beim Abschalten des Magnetfeldes z.B. durch Drehung eines Permanentmagneten oder Unterbrechen eines Stromflusses durch die Spule, kann die Fixierung der magnetischen Partikel und somit der Zellen über den Sensoren aufgehoben werden und tote oder geschädigte Zellen können vom Sensor-Array entfernt bzw. abtransportiert werden. Neue, frische Zellen können erneut über dem Sensor-Array fixiert werden, indem z.B. der Stromfluss in der Spule wieder eingeschaltet wird oder der Permanentmagnet wieder in eine Position gebracht wird, in welcher ein Magnetfeld zum Fixieren der magnetischen Partikel bzw. Magnet-Beads hergestellt wird. Somit steht das Sensor-Array mit lebenden Zellen für eine erneute Messung zur Verfügung.

Die mit der Anordnung zur Bestimmung von Zell-Vitalitäten verbundenen Vorteile sind analog den Vorteilen, welche zuvor in Bezug auf das Verfahren zur Bestimmung von Zell-Vitalitäten beschrieben wurden.

Es wird in den Figuren dargestellt:
- Fig. 1: eine Schnittdarstellung durch eine Durchflusszelle mit einer Einrichtung zum Erzeugen eines Magnetfeldes zum Fixieren von magnetischen Partikeln nach dem Stand der Technik, und
- Fig. 2: eine Schnittdarstellung durch eine Durchflusszelle mit einer Einrichtung zum Erzeugen und Ändern eines Magnetfeldes zum Fixieren von magnetischen Partikeln mit Zellen über einem Sensor-Array (nicht erfindungsgemäße Ausführungsform), und
- Fig. 3: eine vergrößerte Darstellung des in Fig. 2 dargestellten Durchflusskanals mit einer gleichmäßigen Schicht von Zellen in einer magnetischen Partikel Matrix mit der Einrichtung zum Erzeugen und Ändern des Magnetfeldes (nicht erfindungsgemäße Ausführungsform), und
- Fig. 4: eine vergrößerte Darstellung der in Fig. 3 dargestellten gleichmäßigen Schicht von Zellen in einer magnetischen Partikel Matrix auf dem Sensor-Array (nicht erfindungsgemäße Ausführungsform).

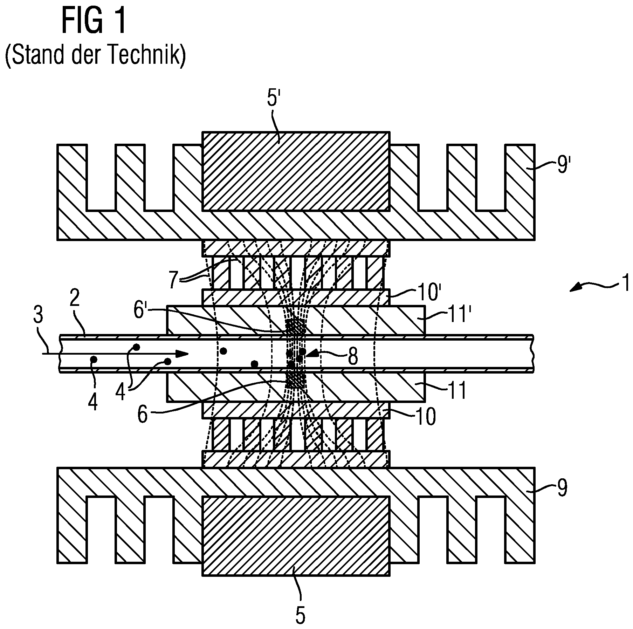

In Fig. 1 ist ein Schnitt durch eine Durchflusszelle 1 nach dem Stand der Technik dargestellt. Die Durchflusszelle 1 umfasst einen Durchflusskanal 2, welcher von einer Flüssigkeit in einer Flussrichtung 3 durchströmt wird. Die Flüssigkeit enthält einzelne magnetische Partikel bzw. Magnet-Beads, an welche z.B. DNA-Fragmente gebunden sein können. Der Durchflusskanal 2 ist von einer Einrichtung zum Erzeugen eines Magnetfeldes 5, 5' umgeben, d.h. oberhalb und unterhalb des Durchflusskanals 2 sind Permanentmagneten angebracht. Über einen Mumetallkörper 6, 6' direkt oberhalb und unterhalb des Durchflusskanals 2 wird das Magnetfeld der Permanentmagneten 5, 5', dargestellt in Fig. 1 mit Hilfe der Magnetfeldlinien 7, auf einen kleinen Bereich innerhalb des Durchflusskanals 2 konzentriert.

Es entsteht im Durchflusskanal 2 ein Ort mit der höchsten Magnetfelddichte 8, an welchem die Magnet-Beads 4 in der Flüssigkeit gesammelt und fixiert werden. Über Kühlkörper 9, 9', welche oberhalb und unterhalb des Durchflusskanals 2 angeordnet sind, und über Peltierelemente 10, 10' in thermischer Verbindung mit Wärmekopplungsplatten 11, 11', ebenfalls jeweils oberhalb und unterhalb des Durchflusskanals 2 angeordnet, kann im Durchflusskanal 2 am Ort mit der höchsten Magnetfelddichte 8 die Temperatur gesteuert bzw. geregelt werden. So können z.B. DNA-Fragmente, gebunden an den Magnet-Beads 4, durch Änderung der Temperatur in Form von zeitlichen Rampen zwischen zwei Temperaturen, über eine PCR (Polymerase-Chain-Reaction) vervielfältigt werden.

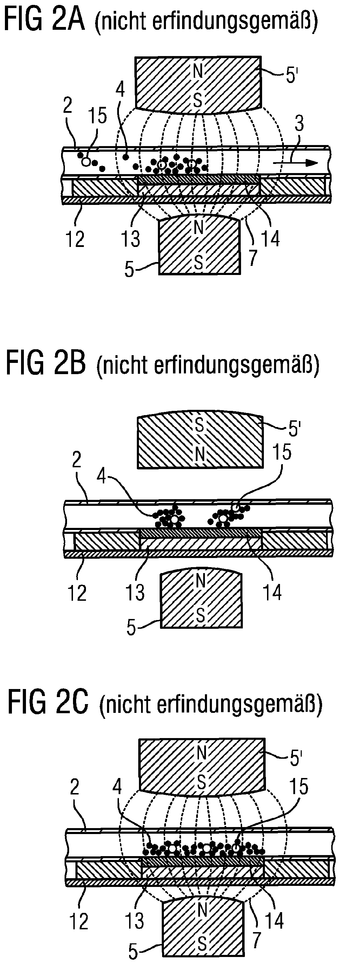

In Fig. 2 (nicht erfindungsgemäße Ausführungsform) ist eine Schnittdarstellung durch eine Anordnung zum Messen der Zell-Vitalität dargestellt. Ein Durchflusskanal 2 ist zwischen zwei Permanentmagneten 5, 5' angeordnet. Eine nicht dargestellte Einrichtung zum Ändern des Magnetfeldes 16, welche z.B. in einem rotierbaren Schrittmotor bestehen kann, ist mit einem der Permanentmagnete 5' verbunden. In Fig. 2 A) ist der Permanentmagnet 5' in einer Position, bei welcher im Inneren des Durchflusskanals 2 ein Magnetfeld vorliegt. Magnetische Partikel 4 im Durchflusskanal werden durch das Magnetfeld im Bereich zwischen dem Permanentmagneten 5 und dem Permanentmagneten 5' fixiert und angesammelt. In diesem Bereich ist ein Chipmodul 12 mit Chip 13 angeordnet, auf welchem sich ein Sensor-Array 14 in fluidischem Kontakt mit dem Durchflusskanal 2 befindet. Durch das Magnetfeld werden somit bei strömender Flüssigkeit die magnetischen Partikel 4 und Zellen 15, welche an magnetische Partikel 4 gebunden sind, über dem Sensor-Array 14 magnetisch fixiert.

In Fig. 2 B) ist die Flüssigkeit in einem ruhenden Zustand und der Permanentmagnet 5' gegenüber der Position von Fig. 2 A) um 90° gedreht, womit im Durchflusskanal 2 kein Magnetfeld verursacht durch den Permanentmagneten 5' vorliegt. Die Feldlinien 7 weisen einen Verlauf auf, welcher nicht vom Permanentmagneten 5 zum Permanentmagneten 5' weisen und den Durchflusskanal 2 durchdringen. Die magnetischen Partikel 4 und Zellen 15 können sich frei über dem Sensor-Array 14 bewegen und umverteilen z.B. durch kreisförmige Flüssigkeitsströmungen über dem Sensor-Array 14 oder durch Diffusion bzw. Konvektion.

In Fig. 2 C) ist die Flüssigkeit in einem ruhenden Zustand und der Permanentmagnet 5' wieder in die Ursprungsposition, wie sie in Fig. 2 A) gezeigt ist, gedreht. Im Bereich über dem Sensor-Array 14 im Durchflusskanal 2 wirkt ein Magnetfeld, dessen Feldlinien 7 einen im Wesentlichen gleichmäßigen Abstand voneinander aufweisen. Die gleichmäßige Feldverteilung über dem Sensor-Array 14 führt zu einer Ausbildung einer im Wesentlichen gleichmäßig dicken Schicht von magnetischen Partikeln 4, in welcher Zellen 15 eingebetet sind. Die magnetischen Partikel 4 bilden eine Art Matrix, in welcher die Zellen 15 über dem Sensor-Array 14 fixiert sind.

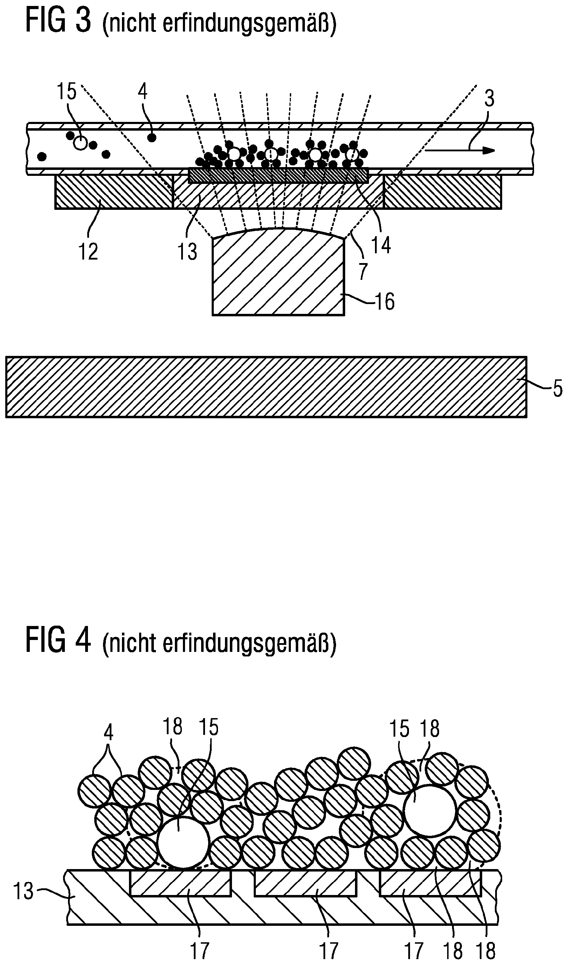

In Fig. 3 (nicht erfindungsgemäße Ausführungsform) ist ein alternatives Beispiel für die Ausbildung eines veränderbaren Magnetfeldes in dem Durchflusskanal 2 über dem Sensor-Array 14 dargestellt. Ein Permanentmagnet 5 ist unterhalb von einem Magnetfeld formenden Element 16 angeordnet. Das Magnetfeld formende Element 16 kann z.B. aus einem magnetisierbaren Eisen bestehen und weist eine äußere Form auf, welche im Durchflusskanal 2 über dem Sensor-Array 14 zur Ausbildung eines besonders gleichmäßigen Magnetfelds führt. In dem in Fig. 3 dargestellten Beispiel ist das Magnetfeld formende Element 16 auf der Seite, welche in Richtung des Sensor-Arrays 14 weist, abgerundet ausgebildet. Das gleichmäßig ausgebildete Magnetfeld über dem Sensor-Array 14 führt zur Ausbildung einer im Wesentlichen gleich dicken Schicht von magnetischen Partikeln 4 mit eingebetteten Zellen 15 über dem Sensor-Array 14. Der Permanentmagnet 5 kann beweglich angeordnet sein und bei Entfernen des Permanentmagneten 5 vom Magnetfeld formenden Element 16 kann das Magnetfeld im Durchflusskanal "ausgeschaltet" werden. Bei wieder annähern des Permanentmagneten 5 an das Magnetfeld formende Element 16 kann das Magnetfeld im Durchflusskanal wieder "eingeschaltet" werden. Alternativ zum Permanentmagneten 5 und/oder dem Magnetfeld formenden Element 16 können elektrische Spulen nahe dem Sensor-Array 14 angeordnet sein, welche bei Stromfluss durch die Spulen ein steuer- oder regelbares Magnetfeld erzeugen.

In Fig. 4 (nicht erfindungsgemäße Ausführungsform) ist eine vergrößerte Darstellung der in den Fig. 2 C) und 3 gezeigten, im Wesentlichen gleichmäßig dicken Schicht magnetischer Partikel 4 mit eingebetteten Zellen 15 über dem Sensor-Array 14 dargestellt. Es sind in der Matrix von magnetischen Partikeln 14 immer Zellen vorhanden, welche mit einem räumlichen Abstand zum nächstgelegenen Sensor 17 angeordnet sind. Dies stellt sicher, dass diese Zellen 15 nicht direkt auf dem Sensor 17 aufwachsen und ein Flüssigkeitsfilm zwischen diesen Zellen 15 und den Sensoren 17 besteht bzw. angeordnet ist. Dadurch werden zuverlässige elektrochemische Messungen der Zell-Vitalität mit den Sensoren 17 erst möglich. Die Zellen 15 können nicht wandern, da sie in der Matrix magnetischer Partikel eingebettet und fixiert sind. Bei einer Ausbildung der Schicht von magnetischen Partikeln 4 mit Zellen 15 mit einer Dicke im Bereich von Mikrometern und einer Anordnung der Sensoren 17 in einer Array-Form mit einem Abstand zwischen den Sensoren 17 im Bereich von Mikrometern jeweils zum nächsten Nachbarn, ist sichergestellt, dass der Bereich der Änderung einer Messgröße 18 durch die Zellen 15 in der Umgebung der Zellen 15 in Kontakt mit wenigstens einem Sensor 17 steht. Bei dem Bereich der Änderung einer Messgröße 18 in der Umgebung von Zellen 15 kann es sich z.B. um die Diffusionslänge von Sauerstoff handeln. Lebende Zellen 15 verbrauchen bei ihrem Stoffumsatz Sauerstoff und die Änderung der Sauerstoffkonzentration kann im Bereich 18 durch die Sensoren 17 gemessen werden.

Sind die Zellen durch die Messung geschädigt oder abgestorben, so können diese einfach durch Abschalten des Magnetfeldes und Einschalten eines Flüssigkeitsstromes abtransportiert werden. Es kann eine neue Schicht magnetischer Partikel 4 mit frischen, lebenden Zellen 15 über dem Sensor-Array 14 ausgebildet werden und die Anordnung kann für eine weitere Messung zur Verfügung stehen. Dadurch ist eine regenerierbare SensorAnordnung, welche intervallartig über längere Zeiten wie z.B. Tage, Wochen oder Monate Messungen durchführen kann, gegeben.

## Patentansprüche

1. Verfahren zur Bestimmung von Zell-Vitalitäten mit den Schritten
- Binden von lebenden Zellen (15) an magnetische Partikel (4), und
- Aufbringen der magnetischen Partikel (4) mit gebundenen Zellen (15) auf einem Sensor-Array (14), und
- gleichmäßige Verteilung der magnetischen Partikel (4) mit den gebundenen Zellen (15) über dem Sensor-Array (14), und
- magnetische Fixierung der magnetischen Partikel (4) mit den gebundenen Zellen (15) über dem Sensor-Array (14), und
- Aufbringen von Substanzen zur Aufrechterhaltung und/oder Verbesserung der Zell-Vitalität auf das Sensor-Array (14), und/oder
- Aufbringen von Substanzen zur Verschlechterung der Zell-Vitalität auf das Sensor-Array (14),
wobei das Sensor-Array (14) mit einer geschlossenen Schicht von magnetischen Partikeln (4) beschichtet wird, bevor eine Schicht mit lebenden Zellen (15) auf dem Sensor-Array (14) gebildet wird,
wobei Hohlräume zwischen den magnetischen Partikeln (4) der geschlossenen Schicht mit Flüssigkeit befüllbar sind.

2. Verfahren nach Anspruch 1, **dadurch gekennzeichnet, dass** von den Substanzen zur Aufrechterhaltung und/oder Verbesserung der Zell-Vitalität Sauerstoff und/oder Nährlösung umfasst wird.

3. Verfahren nach einem der vorhergehenden Ansprüche, **dadurch gekennzeichnet, dass** von den Substanzen zur Verschlechterung der Zell-Vitalität Antibiotika umfasst wird.

4. Verfahren nach einem der vorhergehenden Ansprüche, **dadurch gekennzeichnet, dass** in einem Schritt des Verfahrens eine optimale Temperatur für Zell-Vitalität eingestellt wird, insbesondere 37°C.

5. Verfahren nach einem der vorhergehenden Ansprüche, **dadurch gekennzeichnet, dass** mit Hilfe von Sensoren (17) des Sensor-Arrays (14) elektrochemische und/oder chemische Messungen durchgeführt werden, insbesondere Messungen von Messwerten, welche als Maß für die Zell-Vitalität dienen.

6. Verfahren nach Anspruch 5, **dadurch gekennzeichnet, dass** von Zellen (15) konsumierte Stoffe und/oder Stoffwechselprodukte von Zellen (15) gemessen werden, insbesondere Säuren als pH-Wert und/oder Sauerstoff als pO₂-Wert und/oder Proteine.

7. Verfahren nach einem der vorhergehenden Ansprüche, **dadurch gekennzeichnet, dass** an magnetische Partikel (4) gebundene Zellen (15) über dem Sensor-Array (14) bei Bedarf entfernt werden, insbesondere über eine Manipulation eines Magnetfeldes.

8. Verfahren nach Anspruch 7, **dadurch gekennzeichnet, dass** nach dem Schritt Entfernen von an magnetische Partikel (4) gebundenen Zellen (15) über dem Sensor-Array (14), die Schritte Binden von lebenden Zellen (15) an magnetische Partikel (4), und Aufbringen der magnetischen Partikel (4) mit gebundenen Zellen (15) auf einem Sensor-Array (14), und gleichmäßige Verteilung der magnetischen Partikel (4) mit den gebundenen Zellen (15) über dem Sensor-Array (14), und magnetische Fixierung der magnetischen Partikel (4) mit den gebundenen Zellen (15) über dem Sensor-Array (14), und Aufbringen von Substanzen zur Aufrechterhaltung und/oder Verbesserung der Zell-Vitalität auf das Sensor-Array (14), und/oder Aufbringen von Substanzen zur Verschlechterung der Zell-Vitalität auf das Sensor-Array (14), wiederholt werden, wobei das Sensor-Array (14) mit einer geschlossenen Schicht von magnetischen Partikeln (4) beschichtet wird, bevor eine Schicht mit lebenden Zellen (15) auf dem Sensor-Array (14) gebildet wird.

9. Anordnung zur Bestimmung von Zell-Vitalitäten, insbesondere zur Durchführung des Verfahrens nach einem der vorhergehenden Ansprüche, mit einem Sensor-Array (14) aufgebaut aus Sensoren (17), welche ausgebildet sind mit einem Fluid in direktem fluidischen Kontakt zu stehen, und mit einer Einrichtung zum Erzeugen eines Magnetfeldes (5) über dem Sensor-Array (14), **dadurch gekennzeichnet, dass** auf dem Sensor-Array (14) eine Schicht ausgebildet ist, welche magnetische Partikel (4) und lebende Zellen (15) umfasst, und dass zwischen der Schicht mit den lebenden Zellen (15) und den Sensoren (17) des Sensor-Arrays (14) wenigstens eine geschlossene Schicht von magnetischen Partikeln (4) angeordnet ist, wobei Hohlräume zwischen den magnetischen Partikeln (4) der geschlossenen Schicht mit Flüssigkeit befüllbar sind.

10. Anordnung nach Anspruch 9, **dadurch gekennzeichnet, dass** lebende Zellen (15) in einer Matrix von magnetischen Partikeln (4) in der Schicht auf dem Sensor-Array (14) eingebettet sind.

11. Anordnung nach Anspruch 9 oder 10, **dadurch gekennzeichnet, dass** die Schicht auf dem Sensor-Array (14), umfassend magnetische Partikel (4) und lebende Zellen (15), eine im Wesentlichen gleiche Dicke über dem Bereich des Sensor-Arrays (14) aufweist, insbesondere eine Dicke im Bereich von 10 bis 1000 Mikrometern.

12. Anordnung nach einem der Ansprüche 9 bis 11, **dadurch gekennzeichnet, dass** die Anordnung eine Durchflusszelle (1) mit einem Träger umfasst, wobei auf einer Oberfläche des Trägers in fluidischer Verbindung mit der Durchflusszelle (1) das Sensor-Array (14) angeordnet ist.

13. Anordnung nach einem der Ansprüche 9 bis 12, **dadurch gekennzeichnet, dass** die Sensoren (17) des Sensor-Arrays (14) elektrochemische Sensoren (17) sind, insbesondere Mikrosensoren mit einem Gesamtplatzverbrauch eines Sensors (17) an der Oberfläche des Sensor-Arrays (14) im Bereich von Mikrometern.

14. Anordnung nach einem der Ansprüche 9 bis 13, **dadurch gekennzeichnet, dass** wenigstens eine Einrichtung zum Ändern des Magnetfeldes (16) umfasst ist, insbesondere eine Spuleneinrichtung und/oder eine Einrichtung zum Bewegen von Permanentmagneten.

## Claims

1. Method for determining cell vitalities with the steps
- binding of living cells (15) onto magnetic particles (4), and
- application of the magnetic particles (4) with bound cells (15) onto a sensor array (14), and
- uniform distribution of the magnetic particles (4) with the bound cells (15) over the sensor array (14), and
- magnetic immobilization of the magnetic particles (4) with the bound cells (15) over the sensor array (14), and
- application of substances for maintaining and/or improving the cell vitality onto the sensor array (14), and/or
- application of substances for worsening the cell vitality onto the sensor array (14),
wherein the sensor array (14) is coated with a closed layer of magnetic particles (4) before a layer with living cells (15) is formed on the sensor array (14), wherein cavities between the magnetic particles (4) of the closed layer are fillable with the liquid.

2. Method according to Claim 1, **characterized in that** the substances for maintaining and/or improving the cell vitality comprise oxygen and/or nutrient solution.

3. Method according to one of the previous claims, **characterized in that** the substances for worsening the cell vitality comprise antibiotics.

4. Method according to one of the previous claims, **characterized in that** in one step of the method an optimal temperature for cell vitality is set, in particular 37°C.

5. Method according to one of the previous claims, **characterized in that** by means of sensors (17) of the sensor array (14), electrochemical and/or chemical measurements are performed, in particular measurements of quantities which serve as a measure of the cell vitality.

6. Method according to Claim 5, **characterized in that** substances consumed by cells (15) and/or metabolic products of cells (15) are measured, in particular acids as pH and/or oxygen as pO₂ and/or proteins.

7. Method according to one of the previous claims, **characterized in that** cells (15) bound onto magnetic particles (4) over the sensor array (14) are removed as required, in particular by a manipulation of a magnetic field.

8. Method according to Claim 7, **characterized in that** after the step removal of cells (15) bound onto magnetic particles (4) over the sensor array (14), the steps binding of living cells (15) onto magnetic particles (4), and application of the magnetic particles (4) with bound cells (15) onto a sensor array (14), and uniform distribution of the magnetic particles (4) with the bound cells (15) over the sensor array (14), and magnetic immobilization of the magnetic particles (4) with the bound cells (15) over the sensor array (14), and application of substances for maintaining and/or improving the cell vitality onto the sensor array (14), and/or application of substances for worsening the cell vitality onto the sensor array (14) are repeated, wherein the sensor array (14) is coated with a closed layer of magnetic particles (4) before a layer with living cells (15) is formed on the sensor array (14).

9. Assembly for determining cell vitalities, in particular for performing the method according to one of the previous claims, with a sensor array (14) composed of sensors (17), which are configured to be in direct fluidic contact with a fluid, and with a device for creating a magnetic field (5) over the sensor array (14), **characterized in that** on the sensor array (14) a layer is formed which comprises magnetic particles (4) and living cells (15) and **in that** between the layer with the living cells (15) and **in that** between the layer with the living cells (15) and the sensors (17) of the sensor array (14) at least one closed layer of magnetic particles (4) is located, wherein cavities between the magnetic particles (4) of the closed layer are fillable with liquid.

10. Assembly according to Claim 9, **characterized in that** living cells (15) are embedded in a matrix of magnetic particles (4) in the layer on the sensor array (14).

11. Assembly according to Claim 9 or 10, **characterized in that** the layer on the sensor array (14), comprising magnetic particles (4) and living cells (15) has an essentially equal thickness over the region of the sensor array (14), in particular a thickness in the range from 10 to 1000 micrometers.

12. Assembly according to one of Claims 9 to 11, **characterized in that** the assembly comprises a flow cell (1) with a support, wherein the sensor array (14) is located on one surface of the support in fluid contact with the flow cell (1).

13. Assembly according to one of Claims 9 to 12, **characterized in that** the sensors (17) of the sensor array (14) are electrochemical sensors (17), in particular microsensors with a total space usage of one sensor (17) on the surface of the sensor array (14) in the micrometer range.

14. Assembly according to one of Claims 9 to 13, **characterized in that** at least one device for changing the magnetic field (16) is comprised, in particular a coil device and/or a device for moving permanent magnets.

## Revendications

1. Procédé pour la détermination de vitalités cellulaires avec les étapes
- de liaison de cellules vivantes (15) à des particules magnétiques (4), et
- d'application des particules magnétiques (4) avec des cellules (15) liées sur un réseau de capteurs (14), et
- de répartition uniforme des particules magnétiques (4) avec les cellules (15) liées sur le réseau de capteurs (14), et
- de fixation magnétique des particules magnétiques (4) avec les cellules (15) liées sur le réseau de capteurs (14), et
- d'application de substances pour le maintien et/ou l'amélioration de la vitalité cellulaire sur le réseau de capteurs (14), et/ou
- d'application de substances pour la détérioration de la vitalité cellulaire sur le réseau de capteurs (14),
dans lequel le réseau de capteurs (14) est revêtu d'une couche fermée de particules magnétiques (4) avant qu'une couche avec des cellules vivantes (15) soit formée sur le réseau de capteurs (14),
dans lequel des cavités entre les particules magnétiques (4) de la couche fermée peuvent être remplies de liquide.

2. Procédé selon la revendication 1, **caractérisé en ce que** l'oxygène et/ou une solution nutritive font partie des substances pour le maintien et/ou l'amélioration de la vitalité cellulaire.

3. Procédé selon l'une des revendications précédentes, **caractérisé en ce que** des antibiotiques font partie des substances pour la détérioration de la vitalité cellulaire.

4. Procédé selon l'une des revendications précédentes, **caractérisé en ce qu'**une température optimale pour la vitalité cellulaire est ajustée dans une étape du procédé, en particulier à 37 °C.

5. Procédé selon l'une des revendications précédentes, **caractérisé en ce que** des mesures électrochimiques et/ou chimiques sont mises en oeuvre à l'aide de capteurs (17) du réseau de capteurs (14), en particulier des mesures de valeurs de mesure, lesquelles servent en tant que mesure pour la vitalité cellulaire.

6. Procédé selon la revendication 5, **caractérisé en ce que** des matières consommées par des cellules (15) et/ou des métabolites de cellules (15) sont mesurés, en particulier des acides sous forme de valeur de pH et/ou l'oxygène sous forme de valeur de pO₂ et/ou des protéines.

7. Procédé selon l'une des revendications précédentes, **caractérisé en ce que** des cellules (15) liées à des particules magnétiques (4) sur le réseau de capteurs (14) sont retirées en cas de besoin, en particulier par l'intermédiaire d'une manipulation d'un champ magnétique.

8. Procédé selon la revendication 7, **caractérisé en ce qu'**après l'étape de retrait de cellules (15) liées à des particules magnétiques (4) sur le réseau de capteurs (14), les étapes de liaison de cellules vivantes (15) à des particules magnétiques (4), et d'application des particules magnétiques (4) avec des cellules (15) liées sur un réseau de capteurs (14), et de répartition uniforme des particules magnétiques (4) avec les cellules (15) liées sur le réseau de capteurs (14), et de fixation magnétique des particules magnétiques (4) avec les cellules (15) liées sur le réseau de capteurs (14), et d'application de substances pour le maintien et/ou l'amélioration de la vitalité cellulaire sur le réseau de capteurs (14), et/ou d'application de substances pour la détérioration de la vitalité cellulaire sur le réseau de capteurs (14) sont réitérées, dans lequel le réseau de capteurs (14) est revêtu d'une couche fermée de particules magnétiques (4) avant qu'une couche avec des cellules vivantes (15) soit formée sur le réseau de capteurs (14).

9. Agencement pour la détermination de vitalités cellulaires, en particulier pour la mise en oeuvre du procédé selon l'une des revendications précédentes, avec un réseau de capteurs (14) constitué de capteurs (17), lesquels sont conçus pour être en contact fluidique direct avec un fluide, et avec un dispositif pour la production d'un champ magnétique (5) sur le réseau de capteurs (14), **caractérisé en ce qu'**une couche est constituée sur le réseau de capteurs (14), laquelle comporte des particules magnétiques (4) et des cellules vivantes (15), et **en ce qu'**au moins une couche fermée de particules magnétiques (4) est agencée entre la couche avec les cellules vivantes (15) et les capteurs (17) du réseau de capteurs (14), dans lequel des cavités entre les particules magnétiques (4) de la couche fermée peuvent être remplies de liquide.

10. Agencement selon la revendication 9, **caractérisé en ce que** des cellules vivantes (15) sont incorporées dans une matrice de particules magnétiques (4) dans la couche sur le réseau de capteurs (14).

11. Agencement selon la revendication 9 ou la revendication 10, **caractérisé en ce que** la couche sur le réseau de capteurs (14), comportant des particules magnétiques (4) et des cellules vivantes (15), possède une épaisseur essentiellement égale sur la zone du réseau de capteurs (14), en particulier une épaisseur comprise dans la gamme allant de 10 à 1 000 micromètres.

12. Agencement selon l'une des revendications 9 à 11, **caractérisé en ce que** l'agencement comporte une cellule à écoulement (1) avec un support, dans lequel le réseau de capteurs (14) est agencé sur une surface du support en liaison fluidique avec la cellule à écoulement (1).

13. Agencement selon l'une des revendications 9 à 12, **caractérisé en ce que** les capteurs (17) du réseau de capteurs (14) sont des capteurs électrochimiques (17), en particulier des microcapteurs avec un encombrement global d'un capteur (17) sur la surface du réseau de capteurs (14) de l'ordre des micromètres.

14. Agencement selon l'une des revendications 9 à 13, **caractérisé en ce qu'**au moins un dispositif pour la modification du champ magnétique (16) est inclus, en particulier un dispositif de bobine et/ou un dispositif pour le déplacement d'aimants permanents.
